# EUROPÄISCHE PATENTSCHRIFT

(11) **EP 3 503 994 B1**
(45) Veröffentlichungstag und Bekanntmachung des Hinweises auf die Patenterteilung: **06.03.2024**
(21) Anmeldenummer: 16758132.1
(22) Anmeldetag: 23.08.2016
(51) Int. Cl.: B01D 61/48, B01D 61/50, B01D 61/52, G01N 27/06, G01M 3/16, G01N 33/18, C02F 1/00, C02F 1/20, C02F 1/42, C02F 1/469, C02F 103/02

(54) **VORRICHTUNG UND VERFAHREN ZUR ELEKTRODEIONISATION EINER FLÜSSIGKEIT**
DEVICE AND METHOD FOR THE ELECTRODEIONIZATION OF A LIQUID
DISPOSITIF ET PROCESSUS POUR L'ÉLÉCTRODÉIONISATION D'UN LIQUIDE

(43) Veröffentlichungstag der Anmeldung: 03.07.2019
(73) Patentinhaber: Swan Analytische Instrumente AG, 8340 Hinwil (CH)
(72) Erfinder: MAURER, Heinrich, 8645 Rapperswil-Jona (CH); PFLEGHART, Achilles, 8340 Hinwil (CH); GATH, Julia, 8046 Zürich (CH)
(74) Vertreter: Troesch Scheidegger Werner AG
(86) Internationale Anmeldenummer: PCT/EP2016/069886
(87) Internationale Veröffentlichungsnummer: WO 2018/036612

(56) Entgegenhaltungen:
- EP-A2- 0 777 120
- EP-A2- 1 038 837
- EP-A2- 1 044 717
- WO-A1-02/14850
- JP-A- 2004 050 017

## Beschreibung

Die vorliegende Erfindung betrifft eine Vorrichtung und ein Verfahren zur Elektrodeionisation einer Flüssigkeit.

Die Elektrodeionisation stellt eine Kombination aus Ionenaustausch und Elektrodialyse dar und dient dem Entfernen von Ionen und ionisierbaren Stoffen aus einer Flüssigkeit, insbesondere Wasser. Ziel ist es meist salzfreies Wasser, auch bekannt als deionisiertes oder vollentsalztes (VE) Wasser zu generieren. Ein weiterer Anwendungsbereich liegt in der Überwachung von Prozesswasserkreisläufen in Kraftwerken wie beispielsweise in Wärmekraftwerken. Um zu überprüfen, ob aus dem mit Meer- oder Oberflächenwasser gespeisten Kühlwasserkreislauf Flüssigkeit über ein Leck in den Prozesswasserkreislauf dringen konnte, findet eine spezifische Leitfähigkeitsmessung des Prozesswassers statt. Da dem Prozesswasser unter anderem Ammoniak und/oder Amine zugesetzt sind, um die Korrosion der Leitungen zu verhindern, weist das Prozesswasser bereits inhärent eine spezifische Leitfähigkeit auf, welche sich nicht wesentlich ändern würde durch das Eindringen von salzhaltigem Kühlwasser, insbesondere von im Kühlwasser gelöstem Natriumchlorid (NaCl). Findet die Leitfähigkeitsmessung jedoch nach einem Kationenaustausch statt, so ist die Menge an zur inhärenten Leitfähigkeit beitragenden Kationen aus den Zusätzen reduziert und anstelle von Natriumchlorid (NaCl) liegt Salzsäure (HCl) vor. Da die spezifische Leitfähigkeit einer identischen Anzahl an HCl-Molekülen deutlich höher ist als die spezifische Leitfähigkeit einer identischen Anzahl an NaCl-Molekülen, kann somit das Eindringen von Kühlwasser in den Prozesswasserkreislauf über einen Anstieg der spezifischen Leitfähigkeit des Prozesswassers nach einem Kationentauscher festgestellt werden. Eine Vorrichtung und ein Verfahren zum Nachweis negativer Ionen in Wasser mit dem Zweck ein Eindringen von Kühlwasser in das Prozesswasser nachweisen zu können, sind beispielsweise in EP1167954B1 beschrieben. Die WO0214850A1 betrifft ein Verfahren und eine Vorrichtung zur kontinuierlichen Ionenüberwachung von wässrigen Lösungen durch Messen der "Kationenleitfähigkeit" und der "Anionenleitfähigkeit".

Die Aufgabe der vorliegenden Erfindung besteht darin, eine verbesserte Vorrichtung und eine verbesserte Methode zur Elektrodeionisation einer Flüssigkeit bereitzustellen. Diese Aufgabe wird gelöst durch eine Vorrichtung zur Elektrodeionisation einer Probenflüssigkeit nach einem der Anspüche 1 oder 2.

Es ergeben sich zwei alternative Verbindungsmöglichkeiten der Kammern miteinander. Einerseits können eine der Öffnungen der Behandlungskammer, die Öffnungen der Anodenkammer und eine der Öffnungen der Kathodenkammer so miteinander verbunden sein, dass die Behandlungskammer wirkverbunden ist mit der Anodenkammer und die Anodenkammer wirkverbunden ist mit der Kathodenkammer. Andererseits können eine der Öffnungen der Behandlungskammer, eine der Öffnungen der Anodenkammer und die Öffnungen der Kathodenkammer so miteinander verbunden sein, dass die Behandlungskammer wirkverbunden ist mit der Kathodenkammer und die Kathodenkammer wirkverbunden ist mit der Anodenkammer.

Bei der Energiequelle handelt es sich beispielsweise um eine Spannungsquelle, insbesondere eine Gleichspannungsquelle, welche das unkomplizierte Anlegen einer Gleichspannung zwischen den Elektroden, sprich der Anode und der Kathode, ermöglicht. Die Anode ist dann mit dem Pluspol und die Kathode mit dem Minuspol der Gleichspannungsquelle verbunden. Bei den Elektroden kann es sich unter anderem um Metalldrähte, Metallgewebe oder Metallplatten handeln, welche auch aus Streckmetall gebildet sein können.

Die Öffnungen einer Kammer können beabstandet voneinander angeordnet sein, insbesondere kann eine der Öffnungen im oberen Drittel der Kammer und die andere Öffnung im unteren Drittel der Kammer angeordnet sein. Möglich ist ebenso, dass sich die eine Öffnung auf der Oberseite der Kammer befindet und die andere Öffnung auf der Unterseite der Kammer angeordnet ist.

Bei der permselektiven Membran handelt es sich um eine physikalische Grenzfläche, welche teilweise beziehungsweise halbdurchlässig ist, sprich bestimmte Substanzen/Stoffe zurück hält, andere Substanzen/Stoffe wiederum passieren lässt. Es handelt sich um eine anionendurchlässige oder eine kationendurchlässige Membran Solch eine Membran ist im Wesentlichen undurchlässig für z.B. Wasser, Gas und auch für Elektronen, lässt jedoch entsprechend Anionen respektive Kationen passieren. Das Gegenion wiederum wird zurück gehalten und kann die Membran nicht durchdringen. Aufgebaut sein kann eine solche permselektive Membran beispielsweise aus einem sulfonierten Tetrafluorethylen-Polymer. Weitere Beispiele für geeignete permselektive Membranen sind unter anderem in den Patentschriften US 4324606 und US 4997567 beschrieben. Bei den Kammern kann es sich um drei räumlich trennbare Einheiten handeln, welche beispielsweise über ein Schnellverschlusssystem anstelle der üblichen Gewindezugstangen zu einer kompakten Einheit verbunden werden können, welche alle drei Kammern umfasst. Die Kammern besitzen beispielsweise eine quaderförmige Grundstruktur, wobei zwei Kantenlängen, wie beispielsweise Höhe und Breite, aller Kammern identisch sind, die Tiefe kann variieren. Dabei kann die sich später mittig befindliche Behandlungskammer sechs Seitenflächen umfassen, wovon zwei sich gegenüberliegende Seitenflächen im Wesentlichen gebildet werden durch je eine permselektive Membran. Die Anodenkammer und die Kathodenkammer weisen jedoch nur fünf im Wesentlichen plane Seitenflächen auf und können so mit der Behandlungskammer verbunden werden, dass ihnen je eine permselektive Membran als sechste Seitenfläche dient. Es kann sich jedoch auch um eine Struktur handeln, welche vergleichbar zu einer teilweise abgedeckten Wanne ausgebildet ist. An zwei sich gegenüberliegenden Seiten umfasst eine solche Wanne eine Anode auf der einen, und eine Kathode auf der anderen Seite. Durch Einsetzen einer Behandlungskammer zwischen Anode und Kathode in den nicht abgedeckten Teil der Wanne kann ebenfalls eine drei Kammern umfassende Vorrichtung gebildet werden. Diese eingesetzte Behandlungskammer ist gefüllt mit Anionenaustauscher bzw. Kationenaustauscher und umfasst zwei gegenüberliegende Seitenflächen, die im Wesentlichen gebildet sind durch je eine permselektive Membran. Die Fläche der permselektiven Membranen ist ausreichend gross, um die Wanne in Richtung der Behandlungskammer flüssigkeitsdicht und gasdicht in räumlich drei getrennte Kammern zu teilen. Die hydraulische Wirkverbindung der Kammern kann beispielsweise durch Leitungen erreicht werden. Eine Öffnung der Behandlungskammer kann über einen Schlauch mit einer Öffnung der Anodenkammer verbunden sein. Die andere Öffnung der Anodenkammer ist wiederum über einen Schlauch mit einer der Öffnungen der Kathodenkammer verbunden. Die Öffnung der Behandlungskammer, welche nicht verbunden ist mit der Anodenkammer, kann als Einlassöffnung für die Probenflüssigkeit dienen. Die Öffnung der Kathodenkammer, welche nicht mit der Anodenkammer verbunden ist, kann als Auslassöffnung für die Probenflüssigkeit dienen.

In der Vorrichtung nach Anspruch 2, sind die eine Öffnung der Behandlungskammer, die Öffnungen der Anodenkammer und die eine Öffnung der Kathodenkammer so miteinander verbunden, dass die zugeführte Probenflüssigkeit in der Behandlungskammer im Wesentlichen in Richtung der Schwerkraft und in der Anodenkammer und der Kathodenkammer im Wesentlichen entgegen der Richtung der Schwerkraft geführt wird.

Erreicht werden kann ein solcher Fluss der Probenflüssigkeit beispielsweise, indem die Probenflüssigkeit der Behandlungskammer zugeführt wird über eine an der Oberseite der Behandlungskammer angeordnete Öffnung, die Behandlungskammer daraufhin im Wesentlichen der Länge nach durchströmt wird und die Probenflüssigkeit aus der Behandlungskammer austritt über eine an der Unterseite der Behandlungskammer angeordnete Öffnung. Die Probenflüssigkeit wird dann mittels einer Leitung über eine Öffnung in der Unterseite der Anodenkammer eingeleitet, durchströmt diese im Wesentlichen der Länge nach, fliesst dabei mindestens teilweise zwischen der Anode und der der Anode zugewandten permselektiven Membran und tritt an der Oberseite der Anodenkammer über eine an der Oberseite angeordnete Öffnung aus der Anodenkammer aus. Mittels einer Leitung wird die Probenflüssigkeit dann über eine Öffnung in der Unterseite der Kathodenkammer in diese eingeführt, durchströmt diese im Wesentlichen der Länge nach, fliesst dabei mindestens teilweise zwischen der Kathode und der der Kathode zugewandten permselektiven Membran und tritt an der Oberseite der Kathodenkammer über eine dort angeordnete Öffnung aus.

Der Länge nach bedeutet im Wesentlichen von der Oberseite zur Unterseite und vice versa. In Bezug auf die permselektiven Membranen findet in den Kammern also kein Fluss der Probenflüssigkeit quer sondern lediglich längs zu den Membranen statt.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, ist ein Leitfähigkeitssensor vor der nicht verbundenen Öffnungen der Behandlungskammer angeordnet.

Ein Leitfähigkeitssensor angebracht vor der Öffnung der Behandlungskammer, welche nicht mit einer der Öffnungen der Anodenkammer verbunden ist, kann dazu dienen die Leitfähigkeit der Probenflüssigkeit vor Eintritt in die Behandlungskammer zu bestimmen. Beispielsweise wird das Wasser zur Dampfproduktion im Allgemeinen alkalisiert, da die Eisenoxid-Schutzschichten auf den Innenoberflächen des Wasser-Dampf Kreislaufes bei hohen pH-Werten schwerer löslich sind und deshalb an Ort und Stelle fixiert bleiben. Dadurch wird das darunter liegende Metall vor weiteren Angriffen durch heisses Wasser und Dampf geschützt. Da die Grössen Leitfähigkeit, pH, und Konzentration des Alkalisierungsmittels zusammenhängen, erlaubt die Messung der Leitfähigkeit von Reinstwasser mit einem Alkalisierungsmittel die Konzentration des Alkalisierungsmittels und den pH der Lösung aus Reinstwasser und dem Alkalisierungsmittel zu berechnen. Typische Leitfähigkeiten von Prozesswässern zur Dampferzeugung bewegen sich im Bereich von 8 Mikrosiemens/cm bis 45 Mikrosiemens/cm. Ein typischer Sensor zur Erfassung der elektrischen Leitfähigkeit von Flüssigkeiten ist im Patent US 2611007 "Temperature Compensating Conductivity Cell" beschrieben. Ein anderes Beispiel eines modernen Leitfähigkeitssensors ist der "Swansensor UP-Con 1000" der Firma SWAN Analytical Instruments.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, ist ein Leitfähigkeitssensor zwischen der Behandlungskammer und der Anodenkammer angeordnet.

Ein Leitfähigkeitssensor angeordnet zwischen der Behandlungskammer und der Anodenkammer, beispielsweise in einer Leitung die besagten Kammern verbindend, kann der Bestimmung der Leitfähigkeit der Probenflüssigkeit nach dem Durchfliessen der Behandlungskammer dienen.

Da die Behandlungskammer mit Anionenaustauscherharz bzw. Kationenaustauscherharz mindestens teilweise gefüllt ist, findet beim Durchfliessen der Behandlungskammer ein Ionenaustausch statt, der abhängig von der Art und der Menge der gelösten Ionen in der Probenflüssigkeit einen Einfluss auf deren Leitfähigkeit hat.

Aufgrund der Trennung des Ionenaustauschers von der Anode und der Kathode durch die permselektiven Membranen, können keine durch Elektrolyse an Anode und Kathode entstandenen Gase in die Behandlungskammer dringen und mit der Probenflüssigkeit zum Leitfähigkeitssensor transportiert werden. So kann ein unverfälschtes Messen der Leitfähigkeit erreicht werden. Da die Messung der Leitfähigkeit stattfindet bevor die Probenflüssigkeit die Elektroden passiert, kann eine Oxidation respektive Reduktion der in der Probenflüssigkeit befindlichen Ionen erst nach der Leitfähigkeitsmessung stattfinden.

Wird die Leitfähigkeit der Probenflüssigkeit vor und nach der Behandlungskammer bestimmt, so bietet dies erweiterte analytische Möglichkeiten. So ist es beispielsweise möglich den pH-Wert der Probenflüssigkeit zu bestimmen.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, ist eine Entgasungseinheit nach dem sich zwischen der Behandlungskammer und der Anodenkammer befindlichen Leitfähigkeitssensor angeordnet. Zwischen der Entgasungseinheit und der Anodenkammer ist wiederum ein weiterer Leitfähigkeitssensor angeordnet.

Gase, welche sich in der Probenflüssigkeit befinden und der Behandlungskammer zugeführt werden, können als Gasblasen, also ungelöst oder in gelöster Form auftreten. Gasblasen stören die Leitfähigkeitsmessung an sich und verursachen erratische Signale. Gelöste Gase, welche dissoziieren, also zumindest teilweise in geladene Teile (Ionen) zerfallen, erhöhen die spezifische Leitfähigkeit der Probenflüssigkeit. Beispielsweise kann in Wasser gelöstes Kohlenstoffdioxid (CO₂) abhängig vom pH-Wert Karbonat- oder Bikarbonat-Ionen und Protonen bilden. Das CO₂ kann durch kochen oder andere physikalische Methoden aus der Probenflüssigkeit ausgetrieben werden. Die Leitfähigkeitsmessung der Probenflüssigkeit nach diesem Entgasen ermöglicht beispielsweise die Bestimmung des CO₂-Gehalts.

Wird nur ein Teil der Probenflüssigkeit durch die Entgasungseinheit geführt und der andere Teil direkt in die Anodenkammer geleitet, so kann das Wiederzuführen der durch die Entgasungseinheit geführten Probenflüssigkeit seriell oder parallel erfolgen, sprich die beiden Teile der Probenflüssigkeit können zusammengeführt werden und dann erst in die Anodenkammer eingeleitet werden, oder die beiden Teile können über getrennte Öffnungen der Anodenkammer parallel zugeführt werden. Es kann auch der gesamte Probenstrom nach der Behandlungskammer erst einen Leitfähigkeitssensor, dann eine Entgasungseinheit und dann einen weiteren Leitfähigkeitssensor auf dem Weg in die Anodenkammer durchfliessen. Es ist auch möglich einen Teilstrom zu verwerfen.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, umfasst mindestens einer der Leitfähigkeitssensoren einen Temperatursensor.

Ist ein Temperatursensor in den Leitfähigkeitssensor integriert, so kann die temperaturkompensierte spezifische Leitfähigkeit der Probenflüssigkeit bestimmt werden.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, umfasst die Vorrichtung mindestens einen Durchflusssensor. Dieser mindestens eine Durchflusssensor kann beispielsweise angeordnet sein an einer der Öffnungen der Behandlungskammer, einer der Öffnungen der Anodenkammer und/oder einer der Öffnungen der Kathodenkammer.

Durchflusssensoren ermöglichen eine kontinuierliche Durchflussmessung und können für eine Verifizierung der Leitfähigkeitsmessung sorgen. Ist ein Durchfluss vorhanden, so ist die gemessene spezifische Leitfähigkeit tatsächlich online gemessen und spiegelt den aktuellen Wert wieder.

Typische Durchflussraten liegen beispielsweise zwischen 2 Litern pro Stunde bis 15 Litern pro Stunde.

In einer erfindungsgemässen Ausführungsform der Vorrichtung nach Anspruch 2, ist der Ionenaustauscher ein Kationenaustauscherharz.

Ein geeignetes stark saures gelförmiges Ionentauscherharz ist beispielsweise das Amberjet-1000-H-L (reg) der Firma Rohm und Haas.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, ist der Anionenaustauscher ein farbindizierender Anionenaustauscher bzw. der Kationenaustauscher ein farbindizierender Kationenaustauscher.

Zum Einsatz kommen kann beispielsweise ein stark saures Kationenaustauscherharz, welches mit einem Säure/Base-Farbindikator eingefärbt ist. Ist das Harz "verbraucht", sprich mit Kationen, wie beispielsweise basischen Ammoniumionen (NH₄⁺) beladen, welche ausgetauscht wurden gegen saure Protonen (H⁺), so steigt der pH-Wert an und das Kationenaustauscherharz respektive der Indikator zeigen dies durch eine reversible Farbänderung an. Wird das Harz regeneriert, erscheint wieder die ursprüngliche Farbe. Abweichungen von der für das System typischen Farbverteilung können beispielsweise ein Hinweis auf eine Dysfunktion sein.

Ein Beispiel für ein geeignetes Kationentauscherharz mit Indikator ist der Typ Lewatit S 100 G1 (reg) der Firma Lanxess.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, ist die Behandlungskammer mindestens teilweise transparent ausgebildet, insbesondere entlang der Öffnungen der Behandlungskammer.

Kann in die Behandlungskammer gesehen werden, so können beispielsweise in die Behandlungskammer eingedrungene Verunreinigungen wahrgenommen werden. Auch können Eisenoxid-Ablagerungen auf dem Ionenaustauscher festgestellt werden. Befindet sich ein farbindiz-ierender Ionenaustauscher in der Behandlungskammer, so kann dessen Zustand überprüft werden und bei Bedarf beispielsweise ein Austausch des verbrauchten Ionenaustauschers, der gesamten Behandlungskammer oder sonstige geeignete Massnahmen erfolgen.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, umfasst die Vorrichtung einen optischen Sensor. Der optische Sensor kann zum Überwachen des Ionenaustauschers genutzt werden.

Anstelle der Kontrolle durch das menschliche Auge, kann auch ein optischer Sensor, welcher z.B. eine spektral selektive Reflexionsmessung ausführen kann, so angebracht sein, dass er die Farbe und somit die Güte eines sich in der Behandlungskammer befindlichen farbindizierenden Ionenaustauschers überwachen kann. Wird beispielsweise ein bestimmter kritischer Wert überschritten, so kann eine mit dem optischen Sensor verbundenen Messelektronik einen Alarm auslösen oder eine Steuerelektronik kann den Elektrodeionisationsvorgang oder/und den Probenfluss unterbrechen, da eine einwandfreie Funktion der Vorrichtung nicht mehr garantiert werden kann. Auch ist eine Steuerelektronik denkbar, die einen automatischen Austausch des verbrauchten Ionenaustauschers auslöst.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, umfasst die Vorrichtung eine Messelektronik. Die Messelektronik erfasst und verarbeitet mindestens ein Signal von mindestens einem der Leitfähigkeitssensoren, mindestens ein Signal von mindestens einem der Temperatursensoren, mindestens ein Signal von mindestens einem der Durchflusssensoren, mindestens ein Signal von mindestens dem optischen Sensor, eine Spannung der Energiequelle und/oder einen Strom der Energiequelle.

Eine solche Messelektronik ermöglicht das Aufzeichnen und/oder Auswerten aller von der Vorrichtung umfassten Sensoren. Selbiges gilt für die Kenngrössen Spannung und Strom der Energiequelle. Die Messelektronik kann in ein Panel integriert sein, welches das Ablesen der gemessenen Werte ermöglicht und eine Schnittstelle zu einem externen Computer oder zu einem Datenträger umfassen. Die Messelektronik kann beispielsweise einen Alarm auslösen, sobald von einem Leitfähigkeitssensor eine kritische spezifische Leitfähigkeit der Probenflüssigkeit vor oder nach dem Kationentausch oder nach der Entgasung übermittelt wird. Die Messelektronik kann gekoppelt sein mit einer Steuerelektronik, die entsprechend den über die Messelektronik zugeführten Signalen steuernd auf die Vorrichtung einwirkt. Beispielsweise kann so bei einem erhöhten Durchfluss oder einer angestiegenen Eingangsleitfähigkeit die Spannung zwischen Anode und Kathode erhöht werden, um mehr Protonen an der Anode zu generieren, die wiederum durch die permselektive Membran in die Behandlungskammer wandern und dort den erhöhten Bedarf an Protonen zum Regenerieren des stärker belasteten Ionenaustauschers sicher stellen.

Eine Speisung der Vorrichtung zur Elektrodeionisation ist über eine gemeinsame Energiequelle möglich, die in der Messelektronik integriert sein kann.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, umfasst mindestens eine der Öffnungen der Behandlungskammer eine Filtereinheit.

Eine Filtereinheit angeordnet vor der Öffnung der Behandlungskammer, welche als Einlass für die Probenflüssigkeit dient, verhindert beziehungsweise verringert das Eindringen von Verschmutzungen in die Behandlungskammer. Ebenfalls verhindert sie das Ausschwemmen des Ionentauscherharzes bei aussergewöhnlichen Betriebszuständen des Kraftwerks, etwa beim Herauffahren und Abfahren des Dampferzeugers. Ohne Filtereinheit könnte nämlich auch Wasser und damit auch Ionentauscherharz durch die Einlauföffnung der Vorrichtung zurückgesaugt werden. Eine Filtereinheit vor der Öffnung der Behandlungskammer, welche wirkverbunden ist mit einer Öffnung der Anodenkammer, verhindert das Auswaschen von Ionenaustauscher aus der Behandlungskammer mit der Probenflüssigkeit. Bei der Filtereinheit kann es sich beispielsweise um Filterplatten aus gesintertem Polyethylen handeln, insbesondere aus ultrahochmolekularem gesintertem Polyethylen. Die Porenweite der Filtereinheit kann beispielsweise zwischen 5% und 50% des minimalen Durchmessers des Ionenaustauschers liegen, um den Ionenaustauscher zuverlässig zurückzuhalten.

Typische monodisperse Ionentauscherharze weisen beispielsweise einen Kugeldurchmesser von 0.65 mm mit einem Streubereich von +/- 0.05 mm auf.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, umfasst die Vorrichtung mindestens eine ionenleitende Membran. Hierbei kann es sich beispielsweise um eine weitere permselektive Membran handeln, sofern die ionenleitende Membran vergleichbar oder identisch zu den permselektiven Membranen ist, die die Anodenkammer und die Kathodenkammer von der Behandlungskammer trennen. Diese kann angeordnet sein in der Anodenkammer zwischen der Anode und der ihr zugewandten permselektiven Membran oder in der Kathodenkammer zwischen der Kathode und der ihr zugewandten permselektiven Membran. Es kann auch entsprechend je eine ionenleitende Membran in der Anodenkammer und in der Kathodenkammer angeordnet sein.

Eine solche ionenleitende Membran schützt die permselektive Membran vor an den Elektroden entstehenden, teilweise aggressiven Stoffen, wie beispielsweise naszierendem Sauerstoff oder Ozon. Ebenfalls schützt die ionenleitende Membran die permselektive Membran vor einer Verletzung durch scharfe Kanten, wie sie bei Elektroden aus Streckmetall oder Metallsieben auftreten können.

In einer erfindungsgemässen Ausführungsform der Vorrichtung, welche mit jeder der bereits genannten Ausführungsformen und mit jeder der noch zu nennenden Ausführungsformen kombiniert werden kann, sofern nicht im Widerspruch dazu, ist die Behandlungskammer der Vorrichtung austauschbar.

Zur Verwendung in der erfindungsgemässen Vorrichtung zur Elektrodeionisation einer Probenflüssigkeit wird eine Behandlungskammer bereitgestellt, die eine im Wesentlichen quaderförmige Grundstruktur aufweist. Die Behandlungskammer umfasst zwei voneinander beabstandete Öffnungen und zwei sich gegenüberliegende Seitenflächen, welche im Wesentlichen gebildet werden durch je eine permselektive Membran.

Die Öffnungen können beabstandet voneinander angeordnet sein, z.B. kann eine Öffnung im oberen Teil der Behandlungskammer und eine Öffnung im unteren Teil der Behandlungskammer angeordnet sein. Mit oberem Teil oder Oberteil und unterem Teil oder Unterteil einer Kammer ist, wenn nicht anderes definiert, das untere Drittel und das obere Drittel der Kammer gemeint. Auch kann eine der Öffnungen an der Oberseite und eine an der Unterseite der Behandlungskammer angeordnet sein. Die nicht von den permselektiven Membranen gebildeten Flächen der quaderförmigen Grundstruktur werden beispielsweise durch Kunststoffplatten gebildet, insbesondere mechanisch feste Kunststoffplatten.

In einer Ausführungsform der Behandlungskammer, welche mit jeder der noch zu nennenden Ausführungsformen kombiniert werden kann, sofern nicht im Widerspruch dazu, umfassen die sich gegenüberliegenden Seitenflächen je einen im Wesentlichen rechteckigen Rahmen, welcher einen Teil der Grundstruktur bildet und auf welchem eine permselektive Membran adhäsiv aufgebracht ist.

Die quaderförmige Grundstruktur kann aufgebaut sein aus zwei parallel zueinander ausgerichteten Rahmen, die wasser- und gasdicht verbunden sind mit vier, die quaderförmige Grundstruktur vervollständigenden Platten.

Die Rahmen und Platten können beispielsweise aus Kunststoff sein. Gas- und wasserdicht an den Rahmen angebracht, insbesondere durch Kleben oder Laminieren, ist je eine permselektive Membran, welche den vom Rahmen eingeschlossenen Hohlraum vollständig abdeckt. Um beispielsweise überschüssigen Kleber aufzunehmen, kann der Rahmen eine Nut aufweisen. Die Nut kann umlaufend sein und so angeordnet sein, dass sie sich ausserhalb der Klebestelle befindet aber trotzdem noch von der permselektive Membran überlappt wird.

In einer Ausführungsform der Behandlungskammer, welche mit jeder der bereits genannten Ausführungsformen und mit jeder der noch zu nennenden Ausführungsformen kombiniert werden kann, sofern nicht im Widerspruch dazu, ist benachbart zu einer der Öffnungen eine weitere Öffnung zum Einfüllen von Ionenaustauscher und/oder eine weitere Öffnung zum Entgasen angebracht. Insbesondere ist mindestens eine dieser weiteren Öffnungen wasser- und gasdicht verschliessbar.

Die weitere Öffnung beziehungsweise die weiteren Öffnungen können angeordnet sein im oberen Drittel der Behandlungskammer, insbesondere an der Oberseite der Behandlungskammer, und können wasser- und gasdicht (wieder-)verschliessbar und/oder anschliessbar sein.

In einer Ausführungsform der Behandlungskammer, welche mit jeder der bereits genannten Ausführungsformen und mit jeder der noch zu nennenden Ausführungsformen kombiniert werden kann, sofern nicht im Widerspruch dazu, ist mindestens eine der Öffnungen durch eine Filtereinheit abgedeckt. Die Filtereinheit kann in der Öffnung selbst angeordnet sein, direkt auf der Öffnung, oder auf mindestens einem Stützelement aufliegt.

Die Öffnung zum Einlassen der Probenflüssigkeit und die Öffnung zum Entgasen können beispielsweise über getrennte Filtereinheiten oder über eine gemeinsame Filtereinheit abgedeckt sein. Eine Filtereinheit kann sich sowohl in der Öffnung selbst befinden, als auch auf der Öffnung aufliegen und sich dabei sowohl innerhalb als auch ausserhalb der Behandlungskammer befinden. Eine sich in der Unterseite der Behandlungskammer befindliche Öffnung zum Auslassen der Probenflüssigkeit kann beispielsweise von einer Filtereinheit abgedeckt sein, die sich innerhalb der Behandlungskammer befindet und eine Fläche besitzen, die im Wesentlichen z.B. der Fläche der Unterseite entspricht. Auch kann die Behandlungskammer mit Filtern, beispielsweise Rundfiltern, ausgestattet sein, welche innerhalb der Behandlungskammer im Wesentlichen planparallel zu den permselektiven Membranen angeordnet sind.

Möchte man vermeiden, dass eine solche flächige Filtereinheit direkt auf der Unterseite der Behandlungskammer aufliegt, so kann die Filtereinheit auf mindestens einem Stützelement aufliegen und die Öffnung indirekt abdecken. Durch das indirekte Aufliegen der Filterreinheit kann verhindert werden, dass ein Teil der Filterfläche für den Durchfluss blockiert wird. Als Stützelemente können beispielsweise ein oder mehrere durchbrochene Stützringe, spiralförmige Körper oder radiale Kanäle zum Einsatz kommen. An Stelle von Filterplatten aus gesintertem Polyethylen können als Filtereinheit auch feinmaschige Kunststoffnetze aus Polypropylen, Polyethylen, Polyester und/oder aus einem Fluorpolymer verwendet werden, deren Maschenweite mindestens 50% kleiner als der kleinste Durchmesser der verwendeten Harzkugeln des Ionenaustauscherharzes ist, insbesondere entspricht die Maschenweite 5% bis 50% des kleinsten Durchmessers der Harzkugeln. Geeignete Fluorpolymere sind zum Beispiel PTFE, FEP und/oder ECTFE. Die Netze können beispielsweise adhäsiv oder mit Klemmelementen befestigt sein. Die Netze können ebenfalls auf den erwähnten Stützelementen aufliegen.

In einer Ausführungsform der Behandlungskammer, welche mit jeder der bereits genannten Ausführungsformen und mit jeder der noch zu nennenden Ausführungsformen kombiniert werden kann, sofern nicht im Widerspruch dazu, sind die Aussenseiten der im Wesentlichen durch je eine permselektive Membran gebildeten Seitenflächen mit lösbar fixierten Schutzelementen, insbesondere mit steifen Platten, versehen.

Zum Schutz der Behandlungskammer für Transport und Lagerung können Schutzelemente, wie beispielsweise mechanisch stabile Platten, an den Aussenseiten der im Wesentlichen durch je eine permselektive Membran gebildeten Seitenflächen der quaderförmigen Grundstruktur der Behandlungskammer lösbar angebracht werden. Zum Anbringen der Schutzelemente können z.B. Klebeband oder Montageschlingen verwendet werden. Zusätzlich oder als Alternative kann eine Behandlungskammer zum Schutz auch in einer Tasche, beispielsweise aus Kunststoff, eingeschlossen werden, optional unter Vakuum.

Die Aufgabe wird des Weiteren gelöst durch ein Verfahren zur Elektrodeionisation einer Probenflüssigkeit nach einem der Ansprüche 16 oder 17.

Bei diesem Verfahren finden im Wesentlichen zwei Transportmechanismen gelöster Stoffe statt. Einerseits tritt Konvektion auf, sprich der Transport dieser gelösten Stoffe mit dem Probenflüssigkeitsstrom, andererseits findet Elektromigration statt, sprich die Bewegung elektrisch geladener Teilchen entlang eines elektrischen Feldes. Das elektrische Feld wird erzeugt durch die Spannung zwischen Anode und Kathode. Die Konvektion entspricht dem mindestens teilweisen Durchströmen der Kammern.

Das mindestens teilweise Durchströmen der mindestens teilweise mit Anionenaustauscher bzw. Kationenaustauscher gefüllten Behandlungskammer erfolgt im Wesentlichen in Richtung der Schwerkraft, das mindestens teilweise Durchströmen der Anodenkammer entgegen der Richtung der Schwerkraft und das mindestens teilweise Durchströmen der Kathodenkammer entgegen der Richtung der Schwerkraft.

Kommt es an den Elektroden, sprich in der Anoden- und Kathodenkammer, zur Gasbildung durch Elektrolyse, so kann das entstandene Gas mit dem Probenflüssigkeitsstrom aus der Kammer ausgetragen werden.

In einer erfindungsgemässen Ausführungsform des Verfahrens, welche mit jeder der bereits genannten Ausführungsformen und mit jeder der noch zu nennenden Ausführungsformen kombiniert werden kann, sofern nicht im Widerspruch dazu, umfasst das Verfahren weiter das Messen der spezifischen Leitfähigkeit der Probenflüssigkeit. Das Messen der spezifischen Leitfähigkeit der Probenflüssigkeit findet statt vor dem mindestens teilweisen Durchströmen der mindestens teilweise mit Ionenaustauscher gefüllten Behandlungskammer, begrenzt durch die beiden permselektiven Membranen, mit der Probenflüssigkeit.

In einer erfindungsgemässen Ausführungsform des Verfahrens, welche mit jeder der bereits genannten Ausführungsformen und mit jeder der noch zu nennenden Ausführungsformen kombiniert werden kann, sofern nicht im Widerspruch dazu, umfasst das Verfahren weiter das Messen der spezifischen Leitfähigkeit der Probenflüssigkeit. Das Messen der spezifischen Leitfähigkeit der Probenflüssigkeit findet statt nach dem mindestens teilweisen Durchströmen der mindestens teilweise mit Ionenaustauscher gefüllten Behandlungskammer, begrenzt durch die beiden permselektiven Membranen, mit der Probenflüssigkeit und vor dem mindestens teilweisen Durchströmen der Anodenkammer, befindlich zwischen der Anode und einer der permselektiven Membranen, mit der Probenflüssigkeit.

In einer erfindungsgemässen Ausführungsform des Verfahrens, welche mit jeder der bereits genannten Ausführungsformen und mit jeder der noch zu nennenden Ausführungsformen kombiniert werden kann, sofern nicht im Widerspruch dazu, umfasst das Verfahren weiter das Entgasen mindestens eines Teils der Probenflüssigkeit. Anschliessend findet ein zusätzliches Messen der spezifischen Leitfähigkeit der Probenflüssigkeit vor dem mindestens teilweisen Durchströmen der Anodenkammer, befindlich zwischen der Anode und einer der permselektiven Membranen, mit der Probenflüssigkeit statt.

In einer erfindungsgemässen Ausführungsform des Verfahrens, welche mit jeder der bereits genannten Ausführungsformen kombiniert werden kann, sofern nicht im Widerspruch dazu, umfasst das Verfahren weiter das integrierte, kontinuierliche Messen des Durchflusses der Probenflüssigkeit.

Ausführungsbeispiele der vorliegenden Erfindung werden nachstehend anhand von Figuren noch näher erläutert. Es zeigen
Fig. 1 eine schematisch dargestellte Ausführungsform einer erfindungsgemässen Vorrichtung;
Fig. 2. eine schematische dargestellte Ausführungsform einer erfindungsgemässen Vorrichtung;
Fig. 3. eine schematisch dargestellte Ausführungsform gemäss Fig. 2 einer erfindungsgemässen Vorrichtung mit zwei Leitfähigkeitssensoren;
Fig. 4. eine schematisch dargestellte Ausführungsform gemäss Fig. 3 einer erfindungsgemässen Vorrichtung mit drei Leitfähigkeitssensoren;
Fig. 5. eine schematisch dargestellte Ausführungsform einer Behandlungskammer in perspektivischer Darstellung;
Fig. 6 eine weitere schematisch dargestellte Ausführungsform einer Behandlungskammer in Seitenansicht;
Fig. 7. die Behandlungskammer gemäss Fig. 5 in Seitenansicht;
Fig. 8. eine schematisch dargestellte Ausführungsform einer erfindungsgemässen Vorrichtung mit auswechselbarer Behandlungskammer in perspektivischer Ansicht.

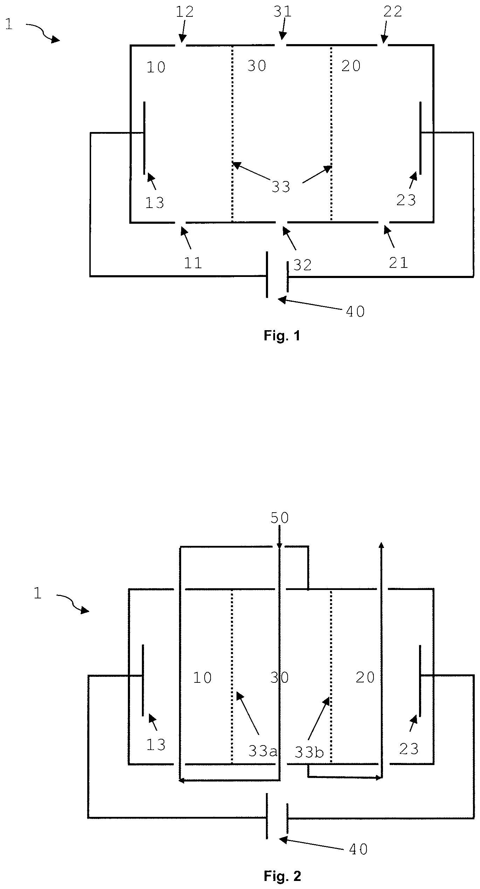

In Figur 1 ist schematisch eine Ausführungsform einer erfindungsgemässen Vorrichtung zur Elektrodeionisation einer Probenflüssigkeit dargestellt.

Die Vorrichtung 1 umfasst einen Anodenraum 10 mit einer Anode 13 und zwei Öffnungen 11,12, eine Kathodenkammer 20 mit einer Kathode 23 und zwei Öffnungen 21,22 und eine Behandlungskammer 30 mit zwei Öffnungen 31,32. Die Behandlungskammer 30 befindet sich zwischen der Anodenkammer 10 und der Kathodenkammer 20 und ist mit Ionenaustauscher befüllt. Die Kammern sind durch permselektive Membranen 33 räumlich voneinander getrennt. Die Anode 13 und die Kathode 23 sind wirkverbunden mit einer Energiequelle 40. Die Energiequelle 40 sorgt für eine zwischen Anode und Kathode anliegende Gleichspannung. Eine der Öffnungen 31,32 der Behandlungskammer dient dazu der Vorrichtung 1 respektive der Behandlungskammer 30 die Probenflüssigkeit zuzuführen, wohingegen eine der Öffnungen 21,22 der Kathodenkammer 20 dazu dient die Probenflüssigkeit aus der Vorrichtung 1 respektive der Kathodenkammer 20 auszuführen. Die restlichen Öffnungen können dazu dienen eine Wirkverbindung zwischen den Kammern herzustellen. Wird beispielsweise Öffnung 31 zum Einlassen der Probenflüssigkeit genutzt, so kann Öffnung 32 zum Erreichen einer Wirkverbindung zwischen Behandlungskammer 30 und Anodenkammer 10 verwendet werden, entweder mittels Öffnung 11 oder 12. Wurde die besagte Wirkverbindung beispielsweise über Öffnung 12 erzeugt, so kann Öffnung 11 mit Öffnung 21 oder 22 verbunden werden, um eine Wirkverbindung zwischen der Anodenkammer 10 und der Kathodenkammer 20 zu erreichen. Werden beispielsweise die Öffnungen 12 und 22 miteinander verbunden, so kann Öffnung 21 zum Auslassen der Probenflüssigkeit aus der Vorrichtung 1 dienen. Alternativ können die Öffnungen auch so miteinander verbunden werden, dass die Behandlungskammer wirkverbunden ist mit der Kathodenkammer und die Kathodenkammer wiederum wirkverbunden ist mit der Anodenkammer. Eine Öffnung der Behandlungskammer 31,32 dient dann ebenso der Zufuhr der Probenflüssigkeit, wohingegen eine Öffnung der Anodenkammer 11,12 dann zum Auslassen der Probenflüssigkeit dient. In der dargestellten Ausführungsform befinden sich alle Öffnungen in der Unterseite oder der Oberseite der Kammern. Es ist auch denkbar, dass die Öffnungen an einer der Seitenwände der Kammern angeordnet sind, beispielsweise je Kammer eine Öffnung im unteren Kammerdrittel und eine Öffnung im oberen Kammerdrittel.

In Figur 2 ist eine erfindungsgemässe Vorrichtung wie in Figur 1 dargestellt anhand derer eine Ausführungsform des erfindungsgemässen Verfahrens erläutert wird. Der Übersichtlichkeit der Darstellung geschuldet sind nicht alle bereits in Figur 1 mittels Bezugszeichen eingeführten Elemente auch in Figur 2 bezeichnet, auch wenn es sich um die identischen Elemente handelt. Dies gilt insbesondere für die dargestellten Öffnungen 11, 12, 21, 22, 31 und 32.

In der abgebildeten beispielhaft dargestellten Vorrichtung 1 wird eine Gleichspannung angelegt zwischen der Anode 13 und der Kathode 23 mittels einer Energiequelle 40. Eine Probenflüssigkeit 50 wird der Behandlungskammer 30 über die Öffnung 31 zugeführt und durchströmt die, mindestens teilweise mit Kationenaustauscherharz gefüllte Behandlungskammer 30 entlang der Öffnungen 31 und 32 in Richtung der Schwerkraft. Da die Öffnungen 32 und 11 wirkverbunden sind, fliesst die Probenflüssigkeit 50, nachdem sie die Behandlungskammer 30 durchströmt hat in die Anodenkammer 10 und durchströmt diese entlang der Öffnungen 11 und 12 entgegen der Richtung der Schwerkraft. Anschliessend tritt die Probenflüssigkeit 50 über die Öffnung 21 in die Kathodenkammer 20 ein und durchströmt diese entlang der Öffnungen 22 und 21 entgegen der Richtung der Schwerkraft. Die Probenflüssigkeit tritt über die Öffnung 22 aus der Kathodenkammer 20 aus.

Die Kammern könnten alternativ auch entgegengesetzt zu ihrer eben beschriebenen Richtung durchflossen werden und die Öffnungen der Kammern können untereinander beliebig wirkverbunden sein, insofern sichergestellt ist, dass die Probenflüssigkeit erst die Behandlungskammer 30, dann die Anodenkammer 10 und dann die Kathodenkammer 20 durchströmt. Die Probenflüssigkeit 50 durchfliesst die Anodenkammer 10 so, dass sie zwischen der Anode 13 und der permselektiven Membran 33a, welche für Kationen durchlässig und der Anode 13 zugewandt ist, fliesst. Die Kathodenkammer 20 wird von der Probenflüssigkeit 50 analog der Anodenkammer 10 in Relation zur Kathode 23 und der Membran 33b durchflossen. Die Behandlungskammer 30 wird von der Probenflüssigkeit 50 parallel zu den Membranen 33a und 33b durchflossen. Die Probenflüssigkeit quert mindestens teilweise das elektrische Feld zwischen der Anode 13 und der Kathode 23 dreimal. Ist die Behandlungskammer mindestens teilweise mit Kationenaustauscherharz gefüllt und handelt es sich bei den permselektiven Membranen um kationendurchlässige Membranen, so kann das beschriebene Verfahren zum Kationenaustausch verwendet werden.

In dem in Figur 2 dargestellten Verfahren treten die im Folgenden näher erläuterten Prozesse auf:
In der Behandlungskammer findet ein Ionenaustausch der in der Probenflüssigkeit gelösten Ionen statt. Ist der Ionenaustauscher, wie in diesem Beispiel, ein Kationenaustauscherharz, so verbleiben die Anionen (z.B. Cl⁻) in der Probenlösung, die Kationen (z.B. NH₄⁺, Na⁺) werden jedoch ausgetauscht durch die mittels des Kationenaustauscherharzes bereitgestellten Kationen (z.B. H⁺). Innerhalb des Kationenaustauscherharzes bewegen sich die Kationen (z.B. NH₄⁺, Na⁺) dann entlang des elektrischen Feldes in Richtung der Kathode und migrieren durch die für Kationen durchlässige, der Kathode zugewandte, permselektive Membran in die Kathodenkammer. Die kationengetauschte Probenflüssigkeit wird von der Behandlungskammer in die Anodenkammer überführt. Dort werden durch Elektrolyse des Wassers in der Probenflüssigkeit Protonen (H⁺) generiert. Diese Protonen (H⁺) können dann in Richtung der Kathode wandern, erst durch die der Anode zugewandte permselektive kationendurchlässige Membran, dann durch die der Kathode zugewandte permselektive kationendurchlässige Membran. Auf ihrem Weg queren die Protonen (H⁺) die Behandlungskammer und stehen dort zur Regeneration des Ionenaustauschers zur Verfügung. Ist das Kationenaustauscherharz regeneriert und sind keine Kationen (mehr) im Probenwasser in der Behandlungskammer vorhanden, migrieren die Protonen weiter in die Kathodenkammer. Die Anionen und das ebenfalls bei der Elektrolyse in der Anodenkammer entstandene Gas (z.B. O₂) werden mit der Probenflüssigkeit in die Kathodenkammer transportiert. In der Kathodenkammer entstehen durch Elektrolyse des Wassers Hydroxidionen (OH⁻) und Gas (z.B. H₂). Die Hydroxidionen neutralisieren die in die Kathodenkammer migrierten Protonen (H⁺) und/oder bilden als Gegenion die entsprechenden Hydroxide (z.B. NH₄OH, NaOH) der in die Kathodenkammer migrierten Kationen (z.B. NH₄⁺, Na⁺) .

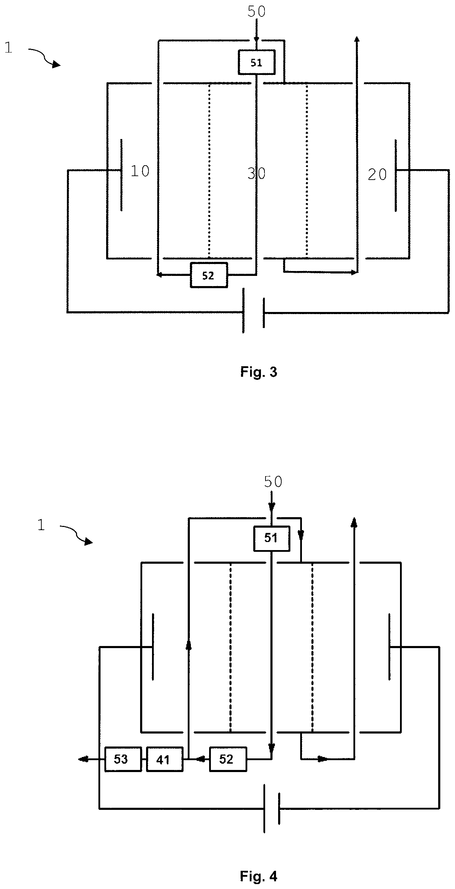

In Figur 3 ist schematisch eine Ausführungsform einer erfindungsgemässen Vorrichtung zur Elektrodeionisation einer Probenflüssigkeit dargestellt. Ebenfalls abgebildet ist der Fliessweg der Probenflüssigkeit.

Bevor die Probenflüssigkeit 50 in die Behandlungskammer 30 eintritt, passiert sie einen vor der Öffnung 31 der Behandlungskammer 30, welche zum Einlass der Probenflüssigkeit 50 dient, angeordneten Leitfähigkeitssensor 51 zum Messen der spezifischen Leitfähigkeit der Probenflüssigkeit 50. Umfasst der Leitfähigkeitssensor 51 einen Temperatursensor, so kann die temperaturkompensierte spezifische Leitfähigkeit bestimmt werden. Ein weiterer Leitfähigkeitssensor 52 ist zwischen der Behandlungskammer 30 und der Anodenkammer 10 angeordnet. Auch dieser Leitfähigkeitssensor 52 kann beim Vorhandensein eines Temperatursensors die temperaturkompensierte spezifische Leitfähigkeit und nicht nur die spezifische Leitfähigkeit messen.

In Figur 4 ist schematisch eine Ausführungsform einer erfindungsgemässen Vorrichtung zur Elektrodeionisation einer Probenflüssigkeit vergleichbar zu Figur 3 dargestellt.

Die abgebildeten Vorrichtungen 1 unterscheiden sich jedoch zu der in Figur 3 dargestellten Vorrichtung durch den weiteren Leitfähigkeitssensor 53. Die Leitfähigkeitsmessung mit diesem Leitfähigkeitssensor 53 unterscheidet sich von den Messungen mit den Leitfähigkeitssensoren 51 und 52 dadurch, dass die "entgaste" (temperaturkompensierte) spezifische Leitfähigkeit bestimmt werden kann. Dies bedeutet, dass dem Leitfähigkeitssensor 53 eine Entgasungseinheit 41 vorgeschalten ist, welche die Probenflüssigkeit entgast. Es ist völlig ausreichend der Entgasungseinheit und dem Leitfähigkeitssensor 53 nur einen Teil der Probenflüssigkeit 50 zuzuführen. Dieser abgezweigte Teil kann dann einfach der restlichen Probenflüssigkeit vor dem Eintritt in die Anodenkammer 10 wieder zugeführt werden oder über eine separate weitere Öffnung ohne vorheriges Vereinen mit der restlichen Probenflüssigkeit in die Anodenkammer 10 geführt werden oder kann einfach verworfen, sprich entsorgt werden. Jedoch kann auch der gesamte ungeteilte Probenstrom sequenziell durch den Leitfähigkeitssensor 51, die Entgasungseinheit 41 und den Leitfähigkeitssensor 53 geleitet werden und dann in die Anodenkammer eingespeist werden.

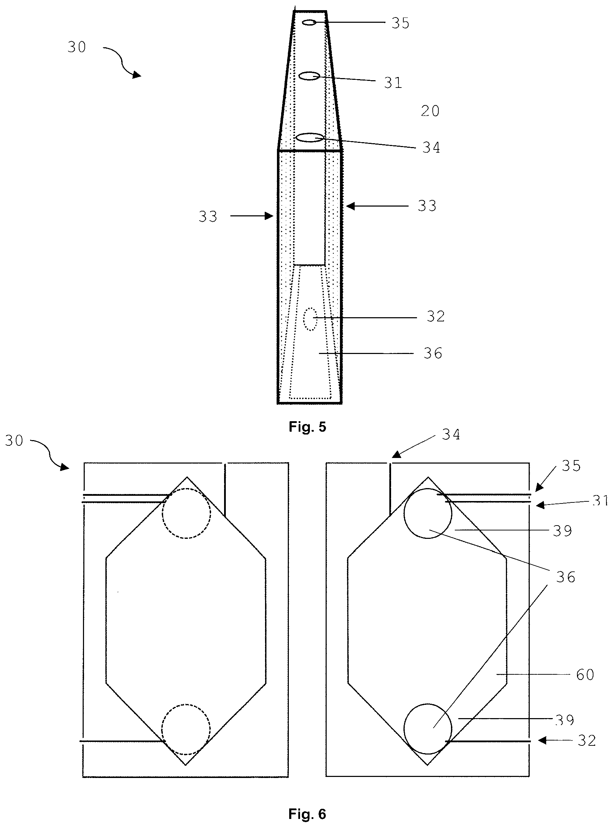

In Figur 5 ist schematisch eine Ausführungsform einer Behandlungskammer zur Verwendung in einer Vorrichtung zur Elektrodeionisation einer Probenflüssigkeit dargestellt.

Bei der Darstellung handelt es sich um eine Frontalansicht einer Behandlungskammer 30 mit einer durchsichtigen Front- und Oberseite. Die Oberseite umfasst eine Öffnung 31, welche in diesem Beispiel zum Einlassen der Probenflüssigkeit 50 dient. Des Weiteren umfasst die Oberseite der Behandlungskammer 30 eine Öffnung 34 zum Einfüllen des Ionenaustauschers und eine Öffnung 35 zum Entgasen der sich in der Behandlungskammer 30 befindlichen Probenflüssigkeit 50. In der Unterseite befindet sich ebenfalls eine Öffnung 32. Diese wird zum Erzeugen einer Wirkverbindung mit der Anodenkammer 10 oder der Kathodenkammer 20 verwendet. Die Öffnung 32 ist durch eine Filtereinheit 36 abgedeckt. Bei der Filtereinheit 36 handelt es sich um eine Filterplatte aus gesintertem Polyethylen deren Porengrösse nur 5 % bis 50 % der Korngrösse des in die Behandlungskammer 30 zu füllenden Ionenaustauschers beträgt. Die Fläche und Kantenlänge der Filterplatte 36 entspricht im Wesentlichen der Fläche und Kantenlänge der Unterseite der Behandlungskammer 30. Die Filterplatte 36 liegt jedoch nicht direkt auf der Unterseite der Behandlungskammer auf, sondern ist abgestützt auf Stützelementen, in diesem Beispiel auf unterbrochenen Stützringen. Alle Öffnungen 31,32,34,35 der Behandlungskammer sind so ausgebildet, dass sie wasserdicht und luftdicht verschlossen werden können.

In Figur 6 ist schematisch eine weitere Ausführungsform einer Behandlungskammer zur Verwendung in einer Vorrichtung zur Elektrodeionisation einer Probenflüssigkeit dargestellt.

Bei der Darstellung handelt es sich um eine Ansicht zweier sich gegenüberliegender Seitenflächen einer Behandlungskammer 30. Zu sehen sind die rechteckigen Rahmen, auf welchen je eine permselektive Membran 33 gas- und wasserdicht adhäsiv aufgebracht ist. Des Weiteren sind zwei planparallel zu den Seitenflächen und sich zwischen diesen befindliche Rundfilter 36 zu sehen, welche einerseits eine Entgasungsöffnung 35 und die Öffnung 31 zum Einlassen der Probenflüssigkeit und andererseits die Öffnung 32 zum Auslassen der Probenflüssigkeit respektive zum Weiterleiten in die Anoden- oder Kathodenkammer abdeckt. An der Oberseite befindet sich zudem eine Öffnung 34 zum Einfüllen des Ionentauschers 60. Auf einer Seitenfläche befindet sich hinter den Filtern 36 je ein Sammelraum 39 für die Probenflüssigkeit. Die Zu- und Abführung der Probenflüssigkeit erfolgt durch eine kurze Bohrung in den jeweiligen Sammelraum 39. Die Achse dieser Bohrungen verläuft im Wesentlichen innerhalb des Rahmens parallel zu den Flächen der permselektiven Membranen und tritt auf der Schmalseite des Rahmens aus.

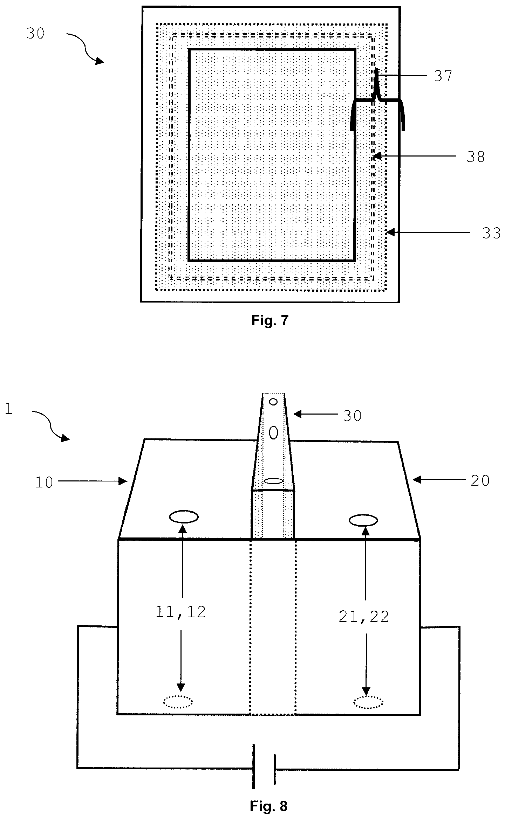

In Figur 7 ist eine schematische Seitenansicht einer Ausführungsform der Behandlungskammer aus Figur 5 dargestellt.

Die gezeigte Seitenfläche der Behandlungskammer 30 umfasst einen Rahmen 37 aus Kunststoff mit einer umlaufenden Nut 28 und einer auf diesem Rahmen 37 adhäsiv aufgebrachten permselektiven Membran 33. Wird die Membran 33 mithilfe eines Klebstoffs an dem Rahmen 37 befestigt, so kann überschüssiger Klebstoff in die Nut 38 fliessen und quillt nicht entlang der Seitenkanten der Membran 33 heraus. Die Membran 33 kann auch mit Hilfe einer Ultraschallschweissung an dem Rahmen 37 befestigt werden.

Alternativ kann eine Seitenfläche einer Behandlungskammer 30 auch aus zwei deckungsgleich angeordneten Rahmen 37 bestehen, zwischen denen eine permselektive Membran 33 befestigt ist, beispielsweise durch Kleben oder Klemmen. Auch können der Rahmen 37 und die Membran 33 durch Laminieren verbunden werden.

In Figur 8 ist schematisch eine Ausführungsform einer erfindungsgemässen Vorrichtung zur Elektrodeionisation einer Probenflüssigkeit dargestellt, welche eine, vergleichbar zu der in Figur 5 dargestellten Behandlungskammer umfasst.

Die Vorrichtung umfasst eine Anodenkammer 10, eine Kathodenkammer 20 und eine austauschbare Behandlungskammer 30, welche passgenau in einen Raum zwischen Anode und Kathode eingebracht wird und so durch ihre Seitenflächen, die permselektiven Membranen 33 umfassend, die eigentliche räumliche Unterteilung der Vorrichtung 1 in drei Kammern bewirkt.

Dargestellt ist insbesondere eine Momentaufnahme des Einsetzens der Behandlungskammer 30. Die geraden, gestrichelten Linien deuten an, wo die Behandlungskammer 30 sich nach dem korrekten Platzieren, zwischen Anode 13 und Kathode 23 befinden wird und mittels Schnellverschlüssen fixiert wird.

Die Anodenkammer 10 und Kathodenkammer 20 sind in diesem Beispiel über eine gemeinsame Unterseite einstückig verbunden. Auf dieser Unterseite können beispielsweise zwei parallele Schienen angebracht sein, welche als Führungselemente für die mit den permselektiven Membranen 33 versehenen Seitenflächen der Behandlungskammer 30 dienen können.

Sinngemäss können auch diejenigen Ausführungsformen und Beispiele, welche für den Kationenaustausch vorgesehen sind, im Rahmen der Erfindung so modifiziert werden, dass sie für den Anionenaustausch verwendet werden können. Wäre beispielsweise ein Anionenaustausch als Form der Elektrodeionisation gewünscht, so wäre die nicht für den Einlass der Probenflüssigkeit vorgesehene Öffnung der Behandlungskammer mit einer Öffnung der Kathodenkammer verbunden, die andere Öffnung der Kathodenkammer wäre mit einer der Öffnungen der Anodenkammer verbunden und die weitere, nicht mit der Kathodenkammer verbundene Öffnung der Anodenkammer würde wiederum als Auslassöffnung für die Probenflüssigkeit dienen.

Ein Leitfähigkeitssensor und optional ein weiterer Leitfähigkeitssensor mit davor angeordneter Entgasungseinheit wären beispielsweise zwischen der Behandlungskammer und der Kathodenkammer angeordnet.

Des Weiteren könnte anstelle von Kationenaustauscher in der Behandlungskammer Anionenaustauscher eingesetzt werden. Die permselektiven Membranen, welche die Behandlungskammer begrenzen wären durchlässig für Anionen anstelle von Kationen.

Weitere sinngemässe Modifikationen im Rahmen der Erfindung können vom Fachmann ohne weiteres erkannt werden, solange sie durch den Umfang der Ansprüche abgedeckt sind.

## Patentansprüche

1. Vorrichtung (1) zur Elektrodeionisation einer Probenflüssigkeit, umfassend:
- eine Anodenkammer (10) umfassend zwei Öffnungen (11, 12) und eine Anode (13);
- eine Kathodenkammer (20) umfassend zwei Öffnungen (21, 22) und eine Kathode (23);
- eine sich zwischen der Anodenkammer (10) und der Kathodenkammer (20) befindlichen Behandlungskammer (30) umfassend zwei Öffnungen (31, 32) und einen Anionenaustauscher, wobei jeweils die Anodenkammer (10) und die Kathodenkammer (20) durch eine anionendurchlässige permselektive Membran (33) von der Behandlungskammer (30) getrennt sind; und
- eine Energiequelle (40) wirkverbunden mit der Anode (13) und der Kathode (23);
wobei
- eine der Öffnungen (31; 32) der Behandlungskammer (30), die Öffnungen (21, 22) der Kathodenkammer (20) und eine der Öffnungen (11; 12) der Anodenkammer (10) so miteinander verbunden sind, dass die Behandlungskammer (30) hydraulisch wirkverbunden ist mit der Kathodenkammer (20) und die Kathodenkammer (20) hydraulisch wirkverbunden ist mit der Anodenkammer (10), wodurch ein mindestens teilweises Durchströmen einer Probeflüssigkeit durch die mindestens teilweise mit Anionenaustauscher gefüllte Behandlungskammer, anschliessend ein mindestens teilweises Durchströmen der Kathodenkammer, und anschliessend ein mindestens teilweises Durchströmen der Anodenkammer ermöglicht wird, **dadurch gekennzeichnet, dass** die eine Öffnung (31; 32) der Behandlungskammer (30), die Öffnungen (21, 22) der Kathodenkammer (20) und die eine Öffnung (11; 12) der Anodenkammer (10) so miteinander verbunden sind, dass die zugeführte Probenflüssigkeit in der Behandlungskammer (30) in Richtung der Schwerkraft und in der Anodenkammer (10) und der Kathodenkammer (20) entgegen der Richtung der Schwerkraft geführt wird.

2. Vorrichtung (1) zur Elektrodeionisation einer Probenflüssigkeit, umfassend:
- eine Anodenkammer (10) umfassend zwei Öffnungen (11, 12) und eine Anode (13);
- eine Kathodenkammer (20) umfassend zwei Öffnungen (21, 22) und eine Kathode (23);
- eine sich zwischen der Anodenkammer (10) und der Kathodenkammer (20) befindlichen Behandlungskammer (30) umfassend zwei Öffnungen (31, 32) und einen Kationenaustauscher, wobei jeweils die Anodenkammer (10) und die Kathodenkammer (20) durch eine kationendurchlässige permselektive Membran (33) von der Behandlungskammer (30) getrennt sind; und
- eine Energiequelle (40) wirkverbunden mit der Anode (13) und der Kathode (23);
wobei
- eine der Öffnungen (31; 32) der Behandlungskammer (30), die Öffnungen (11, 12) der Anodenkammer (10) und eine der Öffnungen (21; 22) der Kathodenkammer (20) so miteinander verbunden sind, dass die Behandlungskammer (30) hydraulisch wirkverbunden ist mit der Anodenkammer (10) und die Anodenkammer (10) hydraulisch wirkverbunden ist mit der Kathodenkammer (20), wodurch ein mindestens teilweises Durchströmen einer Probeflüssigkeit durch die mindestens teilweise mit Kationenaustauscher gefüllte Behandlungskammer, anschliessend ein mindestens teilweises Durchströmen der Anodenkammer und anschliessend ein mindestens teilweises Durchströmen der Kathodenkammer ermöglicht wird, **dadurch gekennzeichnet, dass**
die eine Öffnung (31; 32) der Behandlungskammer (30), die Öffnungen (11, 12) der Anodenkammer (10) und die eine Öffnung (21; 22) der Kathodenkammer (20) so miteinander verbunden sind, dass die zugeführte Probenflüssigkeit in der Behandlungskammer (30) in Richtung der Schwerkraft und in der Anodenkammer (10) und der Kathodenkammer (20) entgegen der Richtung der Schwerkraft geführt wird.

3. Vorrichtung (1) nach einem der Ansprüche 1 bis 2, wobei ein Leitfähigkeitssensor (51) vor der nicht verbundenen Öffnungen (31; 32) der Behandlungskammer (30) angeordnet ist.

4. Vorrichtung (1) nach einem der Ansprüche 1 bis 3, wobei ein Leitfähigkeitssensor (52) zwischen der Behandlungskammer (30) und der Anodenkammer (10) angeordnet ist.

5. Vorrichtung (1) nach Anspruch 4, umfassend eine Entgasungseinheit (41) angeordnet nach dem Leitfähigkeitssensor (52), wobei nach der Entgasungseinheit (41) ein weiterer Leitfähigkeitssensor (53) angeordnet ist.

6. Vorrichtung (1) nach einem der Ansprüche 3 bis 5, wobei mindestens einer der Leitfähigkeitssensoren (51; 52; 53) einen Temperatursensor umfasst.

7. Vorrichtung (1) nach einem der Ansprüche 1 bis 6, umfassend mindestens einen Durchflusssensor angeordnet vor mindestens einem der Folgenden:
- einer der Öffnungen (31; 32) der Behandlungskammer (30)
- einer der Öffnungen (11; 12) der Anodenkammer (10)
- einer der Öffnungen (21; 22) der Kathodenkammer (20).

8. Vorrichtung (1) nach einem der Ansprüche 1 bis 7, wobei der Anionenaustauscher ein farbindizierender Anionenaustauscher bzw. der Kationenaustauscher ein farbindizierender Kationenaustauscher ist.

9. Vorrichtung (1) nach einem der Ansprüche 1 bis 8, wobei die Behandlungskammer (30) mindestens teilweise transparent ausgebildet ist, insbesondere entlang der Öffnungen (31, 32) der Behandlungskammer (30), und wobei die Vorrichtung bevorzugt einen optischen Sensor zum Überwachen des Anionenaustauschers bzw. Kationenaustauschers umfasst.

10. Vorrichtung (1) nach einem der Ansprüche 3 bis 9 umfassend eine Messelektronik, welche mindestens eines der Folgenden:
- Signal von mindestens einem der Leitfähigkeitssensoren
- Signal von mindestens einem der Temperatursensoren
- Signal von mindestens einem der Durchflusssensoren
- Signal von mindestens dem optischen Sensor
- eine Spannung der Energiequelle (40)
- einen Strom der Energiequelle (40)
erfasst und verarbeitet.

11. Vorrichtung (1) nach einem der Ansprüche 1 bis 10, umfassend mindestens eine ionenleitende Membran, angeordnet in mindestens einer der Folgenden:
- Anodenkammer (10) zwischen der Anode (13) und der der Anode (13) zugewandten permselektiven Membran (33)
- Kathodenkammer (20) zwischen der Kathode (23) und der der Kathode (23) zugewandten permselektiven Membran (33).

12. Vorrichtung (1) nach einem der Ansprüche 1 bis 11, wobei die Behandlungskammer (30) austauschbar ist und/oder wobei mindestens eine der Öffnungen (31, 32) der Behandlungskammer (30) eine Filtereinheit umfasst.

13. Vorrichtung nach einem der Ansprüche 1 bis 12 wobei die Behandlungskammer (30) austauschbar ist und eine quaderförmige Grundstruktur aufweist, umfassend zwei voneinander beabstandete Öffnungen (31, 32) und zwei sich gegenüberliegende Seitenflächen, welche gebildet werden durch je eine entsprechende permselektive Membran (33), welche den vom Rahmen gebildeten Hohlraum vollständig abdecken, wobei die sich gegenüberliegenden Seitenflächen je einen rechteckigen Rahmen, einen Teil der Grundstruktur bildend, umfassen, auf welchem eine permselektive Membran (33) adhäsiv gas- und wasserdicht aufgebracht ist, und wobei der Rahmen eine Nut aufweist.

14. Vorrichtung nach einem der Ansprüche 1 bis 12 wobei die Behandlungskammer (30) austauschbar ist und eine quaderförmige Grundstruktur aufweist, umfassend zwei voneinander beabstandete Öffnungen (31, 32) und zwei sich gegenüberliegende Seitenflächen, welche gebildet werden durch je eine anionendurchlässige permselektive Membran (33) im Falle eines Anionenaustauschers oder durch je eine kationendurchlässige permselektive Membran (33) im Falle eines Kationenaustauschers, wobei die sich gegenüberliegenden Seitenflächen je einen rechteckigen Rahmen, einen Teil der Grundstruktur bildend, umfassen, auf welchem die permselektive Membran (33) adhäsiv angebracht ist und wobei benachbart zu einer der Öffnungen (31; 32) eine weitere Öffnung zum Einfüllen von Anionenaustauscher bzw. Kationenaustauscher und/oder eine weitere Öffnung zum Entgasen angebracht ist, wobei bevorzugt mindestens eine der weiteren Öffnungen wasser- und gasdicht verschliessbar ist.

15. Vorrichtung nach einem der Ansprüche 1 bis 12 wobei die Behandlungskammer (30) austauschbar ist und eine quaderförmige Grundstruktur aufweist, umfassend zwei voneinander beabstandete Öffnungen (31, 32) und zwei sich gegenüberliegende Seitenflächen, welche gebildet werden durch je eine permselektive Membran (33), wobei die sich gegenüberliegenden Seitenflächen je einen rechteckigen Rahmen, einen Teil der Grundstruktur bildend, umfassen, auf welchem die permselektive Membrane (33) adhäsiv angebracht ist, und wobei die Aussenseiten der durch die je eine permselektive Membran (33) gebildeten Seitenflächen mit lösbar fixierten Schutzelementen, insbesondere mit steifen Platten, versehen sind.

16. Verfahren zur Elektrodeionisation einer Probenflüssigkeit zur Ausführung in einer Vorrichtung gemäss Anspruch 2, umfassend die Schritte:
- Anlegen einer Spannung an eine räumlich durch zwei kationendurchlässige permselektive Membranen (33a, 33b) getrennte Anode (13) und Kathode (23);
P217173 Neue Ansprüche Ladung mdl. Verhandlung 26.05.2023 Reinschrift
- mindestens teilweise Durchströmen einer mindestens teilweise mit Kationenaustauscher gefüllten Behandlungskammer (30), begrenzt durch die beiden kationendurchlässigen permselektiven Membranen (33a, 33b), mit der Probenflüssigkeit; anschliessend
- mindestens teilweise Durchströmen einer Anodenkammer (10), befindlich zwischen der Anode (13) und einer der kationendurchlässigen permselektiven Membranen (33a), mit der Probenflüssigkeit; anschliessend
- mindestens teilweise Durchströmen einer Kathodenkammer (20), befindlich zwischen der Kathode (23) und der anderen kationendurchlässigen permselektiven Membran (33b), mit der Probenflüssigkeit,
**dadurch gekennzeichnet, dass**
- das mindestens teilweise Durchströmen der mindestens teilweise mit Kationenaustauscher gefüllten Behandlungskammer (30) in Richtung der Schwerkraft erfolgt;
- das mindestens teilweise Durchströmen der Anodenkammer (10) entgegen der Richtung der Schwerkraft erfolgt; und
- das mindestens teilweise Durchströmen der Kathodenkammer (20) entgegen der Richtung der Schwerkraft erfolgt.

17. Verfahren zur Elektrodeionisation einer Probenflüssigkeit zur Ausführung in einer Vorrichtung nach Anspruch 1, umfassend die Schritte:
- Anlegen einer Spannung an eine räumlich durch zwei anionendurchlässige permselektive Membranen (33a, 33b) getrennte Anode (13) und Kathode (23);
- mindestens teilweise Durchströmen einer mindestens teilweise mit Anionenaustauscher gefüllten Behandlungskammer (30), begrenzt durch die beiden anionendurchlässigen permselektiven Membranen (33a, 33b), mit der Probenflüssigkeit; anschliessend
- mindestens teilweise Durchströmen einer Kathodenkammer (20), befindlich zwischen der Kathode (23) und der anderen anionendurchlässigen permselektiven Membran (33b), mit der Probenflüssigkeit; anschliessend
- mindestens teilweise Durchströmen einer Anodenkammer (10), befindlich zwischen der Anode (13) und einer der anionendurchlässigen permselektiven Membranen (33a), mit der Probenflüssigkeit,
**dadurch gekennzeichnet, dass**
- das mindestens teilweise Durchströmen der mindestens teilweise mit Anionenaustauscher gefüllten Behandlungskammer (30) in Richtung der Schwerkraft erfolgt;
- das mindestens teilweise Durchströmen der Anodenkammer (10) entgegen der Richtung der Schwerkraft erfolgt; und
- das mindestens teilweise Durchströmen der Kathodenkammer (20) entgegen der Richtung der Schwerkraft erfolgt.

18. Verfahren nach einem der Ansprüche 16 bis 17, umfassend mindestens einen der weiteren Schritte:
- Messen der spezifischen Leitfähigkeit der Probenflüssigkeit
vor dem Schritt des mindestens teilweisen Durchströmens der mindestens teilweise mit Anionenaustauscher bzw. mit Kationenaustauscher gefüllten Behandlungskammer (30), begrenzt durch die beiden permselektive Membranen (33a, 33b), mit der Probenflüssigkeit;
- Messen der spezifischen Leitfähigkeit der Probenflüssigkeit
nach dem Schritt des mindestens teilweisen Durchströmens der mindestens teilweise mit Anionenaustauscher bzw. der Kationenaustauscher gefüllten Behandlungskammer (30), begrenzt durch die beiden permselektiven Membranen (33a, 33b), mit der Probenflüssigkeit, insbesondere in Kombination mit den weiteren Schritten des Entgasens mindestens eines Teils der Probenflüssigkeit und anschliessend des zusätzlichen Messens der spezifischen Leitfähigkeit
nach dem Schritt des Messens der spezifischen Leitfähigkeit der Probenflüssigkeit;
- integriertes, kontinuierliches Messen des Durchflusses der Probenflüssigkeit.

## Claims

1. Device (1) for electrodeionization of a sample liquid comprising:
- an anode chamber (10) comprising two openings (11, 12) and an anode (13);
- a cathode chamber (20) comprising two openings (21, 22) and a cathode (23);
- a treatment chamber (30) located between the anode chamber (10) and the cathode chamber (20) comprising two openings (31, 32) and anion exchanger, wherein the anode chamber (10) and the cathode chamber (20) are separated each from the treatment chamber (30) by an anion-permeable permselective membrane (33); and
- an energy source (40) operatively connected to the anode (13) and the cathode (23);
wherein
- one of the openings (31; 32) of the treatment chamber (30), the openings (21, 22) of the cathode chamber (20) and one of the openings (11; 12) of the anode chamber (10) are connected to each other such that the treatment chamber (30) is hydraulically operatively connected to the cathode chamber (20) and the cathode chamber (20) is hydraulically operatively connected to the anode chamber (10), whereby an at least partial flow of a sample liquid through the treatment chamber which is at least partially filled with the anion exchanger, then an at least partial flow through the cathode chamber, and then an at least partial flow through the anode chamber is made possible, **characterized in that**
- the one opening (31; 32) of the treatment chamber (30), the openings (21, 22) of the cathode chamber (20) and the one opening (11; 12) of the anode chamber (20) are connected to each other in such a way that the supplied sample liquid in the treatment chamber (30) is guided in the direction of gravity and is guided in the anode chamber (10) and the cathode chamber (20) against the direction of gravity.

2. Device (1) for electrodeionization of a sample liquid comprising:
- an anode chamber (10) comprising two openings (11, 12) and an anode (13);
- a cathode chamber (20) comprising two openings (21, 22) and a cathode (23);
- a treatment chamber (30) located between the anode chamber (10) and the cathode chamber (20) comprising two openings (31, 32) and an cation exchanger, wherein the anode chamber (10) and the cathode chamber (20) are separated each from the treatment chamber (30) by a cation-permeable permselective membrane (33); and
- an energy source (40) operatively connected to the anode (13) and the cathode (23);
wherein
- one of the openings (31; 32) of the treatment chamber (30), the openings (11, 12) of the anode chamber (10) and one of the openings (21; 22) of the cathode chamber (20) are connected to each other such that the treatment chamber (30) is hydraulically operatively connected to the anode chamber (10) and the anode chamber (10) is hydraulically operatively connected to the cathode chamber (20), whereby an at least partial flow of a sample liquid through the treatment chamber which is at least partially filled with the cation exchanger, then an at least partial flow through the anode chamber, and then an at least partial flow through the cathode chamber is made possible, **characterized in that**
- the one opening (31; 32) of the treatment chamber (30), the openings (11, 12) of the anode chamber (10) and the one opening (21; 22) of the cathode chamber (20) are connected to each other in such a way that the supplied sample liquid in the treatment chamber (30) is guided in the direction of gravity and is guided in the anode chamber (10) and the cathode chamber (20) against the direction of gravity.

3. Device (1) according to one of claims 1 to 2, wherein a conductivity sensor (51) is arranged in front of the unconnected openings (31; 32) of the treatment chamber (30) .

4. Device (1) according to one of claims 1 to 3, wherein a conductivity sensor (52) is arranged between the treatment chamber (30) and the anode chamber (10).

5. Device (1) according to claim 4, comprising a degassing unit (41) arranged after the conductivity sensor (52), wherein a further conductivity sensor (53) is arranged after the degassing unit (41).

6. Device (1) according to one of claims 3 to 5, wherein at least one of the conductivity sensors (51; 52; 53) comprises a temperature sensor.

7. Device (1) according to one of claims 1 to 6, comprising at least one flow sensor arranged in front of at least one of the following:
- one of the openings (31; 32) of the treatment chamber (30)
- one of the openings (11; 12) of the anode chamber (10)
- one of the openings (21; 22) of the cathode chamber (20) .

8. Device (1) according to one of claims 1 to 7, wherein the anion exchanger is a color-indicating anion exchanger or the cation exchanger is a color-indicating cation exchanger.

9. Device (1) according to one of claims 1 to 8, wherein the treatment chamber (30) is formed at least partially transparent, in particular along the openings (31, 32) of the treatment chamber (30), and wherein the device comprises preferably an optical sensor for monitoring the anion exchanger or the cation exchanger.

10. Device (1) according to one of claims 3 to 9, comprising an electronic measuring equipment, which records and processes at least one of the following:
- signal from at least one of the conductivity sensors
- signal from at least one of the temperature sensors
- signal from at least one of the flow sensors
- signal from at least the optical sensor
- a voltage of the power source (40)
- a current of the power source (40).

11. Device (1) according to one of claims 1 to 10, comprising at least one ion-conducting membrane, arranged in at least one of the following:
- anode chamber (10) between the anode (13) and the permselective membrane (33) facing the anode (13)
- cathode chamber (20) between the cathode (23) and the permselective membrane (33) facing the cathode (23).

12. Device (1) according to one of claims 1 to 11, wherein the treatment chamber (30) is exchangeable and/or wherein at least one of the openings (31, 32) of the treatment chamber (30) comprises a filter unit.

13. Device (1) according to one of claims 1 to 12, wherein the treatment chamber (30) is exchangeable and has a cuboid base structure comprising two mutually spaced apart openings (31, 32) and two mutually opposite side surfaces, which are formed by a respective permselective membrane (33) each, which covers the hollow space formed by the frame completely, wherein the mutually opposite side surfaces comprise a rectangular frame each, the rectangular frame forming part of the basic structure on which a permselective membrane (33) is adhesively applied in a water-tight and gas-tight manner, and wherein the frame comprises a notch.

14. Device (1) according to one of claims 1 to 12, wherein the treatment chamber (30) is exchangeable and has a cuboid base structure comprising two mutually spaced apart openings (31, 32) and two mutually opposite side surfaces, which are formed by an anion-permeable permselective membrane (33) in case of an anion exchanger or by an cation-permeable permselective membrane in case of a cation exchanger, wherein the mutually opposite side surfaces comprise a rectangular frame each, the rectangular frame forming part of the basic structure on which a permselective membrane (33) is adhesively applied and wherein a further opening for filling anion exchanger or cation exchanger and/or a further opening for degassing is provided adjacent to one of the openings (31; 32), wherein at least one of the further openings is closable in a water-tight and gas-tight manner.

15. Device (1) according to one of claims 1 to 12, wherein the treatment chamber (30) is exchangeable and has a cuboid base structure comprising two mutually spaced apart openings (31, 32) and two mutually opposite side surfaces, which are formed by a respective permselective membrane (33) each, wherein the mutually opposite side surfaces comprise a rectangular frame each, the rectangular frame forming part of the basic structure on which a permselective membrane (33) is adhesively applied and wherein the outer sides of the side surfaces formed by a permselective membrane (33) each are provided with detachably fixed protective elements, in particular with rigid plates.

16. Method for electrodeionization of a sample liquid for implementation in a device according to claim 2, the method comprising the steps of:
- applying a voltage to an anode (13) and cathode (23) spatially separated from each other by two cation-permeable permselective membranes (33a, 33b);
- at least partial flow of the sample liquid through a treatment chamber (30) which is at least partially filled with cation exchanger and is bounded by the two cation-permeable permselective membranes (33a, 33b); then
- at least partial flow of the sample liquid through an anode chamber (10) which is arranged between the anode (13) and one of the cation-permeable permselective membranes (33a); then
- at least partial flow of the sample liquid through a cathode chamber (20) which is arranged between the cathode (23) and the other cation-permeable permselective membrane (33a),
**characterized in that**
- the at least partial flow through the treatment chamber (30), which is at least partially filled with cation exchanger, takes place in the direction of gravity;
- the at least partial flow through the anode chamber (10) takes place against the direction of gravity; and
- the at least partial flow through the cathode chamber (20) takes place against the direction of gravity.

17. Method for electrodeionization of a sample liquid for implementation in a device according to claim 1, the method comprising the steps of:
- applying a voltage to an anode (13) and cathode (23) spatially separated from each other by two anion-permeable permselective membranes (33a, 33b);
- at least partial flow of the sample liquid through a treatment chamber (30) which is at least partially filled with anion exchanger and is bounded by the two anion-permeable permselective membranes (33a, 33b); then
- at least partial flow of the sample liquid through an cathode chamber (20) which is arranged between the cathode (23) and one of the anion-permeable permselective membranes (33a); then
- at least partial flow of the sample liquid through a anode chamber (10) which is arranged between the anode (13) and the other anion-permeable permselective membrane (33a), **characterized in that**
- the at least partial flow through the treatment chamber (30), which is at least partially filled with anion exchanger, takes place in the direction of gravity;
- the at least partial flow through the anode chamber (10) takes place against the direction of gravity; and
- the at least partial flow through the cathode chamber (20) takes place against the direction of gravity.

18. Method according to one of claims 16 to 17, comprising at least one of the further steps of:
- measuring the specific conductivity of the sample liquid
before the step of the at least partial flow of the sample liquid through the treatment chamber (30) which is at least partially filled with anion exchanger or cation exchanger and is bounded by the two permselective membranes (33a, 33b) ;
- measuring the specific conductivity of the sample liquid
after the step of the at least partial flow of the sample liquid through the treatment chamber (30) which is at least partially filled with anion exchanger or cation exchanger and is bounded by the two permselective membranes (33a, 33b), in particular in combination with the further steps of degassing of at least a part of the sample liquid and subsequently additionally measuring the specific conductivity of the sample liquid after the step of measuring the specific conductivity of the sample liquid;
- integrated, continuous measurement of the sample liquid flow rate.

## Revendications

1. Dispositif (1) pour l'électrodéionisation d'un échantillon liquide, comprenant :
- une chambre d'anode (10) comportant deux ouvertures (11, 12) et une anode (13) ;
- une chambre de cathode (20) comportant deux ouvertures (21, 22) et une cathode (23) ;
- une chambre de traitement (30) disposée entre la chambre d'anode (10) et la chambre de cathode (20) et comportant deux ouvertures (31, 32) et un échangeur d'anions, la chambre d'anode (10) et la chambre de cathode (20) étant respectivement séparées de la chambre de traitement (30) par l'intermédiaire d'une membrane permsélective perméable aux anions (33) ; et
- une source d'énergie (40) en liaison active avec l'anode (13) et la cathode (23) ;
dans lequel
- une des ouvertures (31 ; 32) de la chambre de traitement (30), les ouvertures (21, 22) de la chambre de cathode (20) et l'une des ouvertures (11 ; 12) de la chambre d'anode (10) sont reliées entre elles, de sorte que la chambre de traitement (30) est en liaison active hydrauliquement avec la chambre de cathode (20) et que la chambre de cathode (20) est en liaison active hydrauliquement avec la chambre d'anode (10), permettant ainsi à un échantillon liquide de s'écouler au moins partiellement à travers la chambre de traitement remplie au moins en partie du échangeur d'anions, puis de s'écouler au moins partiellement à travers la chambre de cathode, et puis de s'écouler au moins partiellement à travers la chambre d'anode, **caractérisé en ce que**
ladite ouverture (31 ; 32) de la chambre de traitement (30), les ouvertures (21, 22) de la chambre de cathode (20) et ladite ouverture (11 ; 12) de la chambre d'anode (10) sont reliées entre elles de sorte que l'échantillon liquide fourni est guidé dans la chambre de traitement (30) dans le sens de la gravité et dans la chambre d'anode (10) et la chambre de cathode (20) dans le sens contraire à la gravité.

2. Dispositif (1) pour l'électrodéionisation d'un échantillon liquide, comprenant :
- une chambre d'anode (10) comportant deux ouvertures (11, 12) et une anode (13) ;
- une chambre de cathode (20) comportant deux ouvertures (21, 22) et une cathode (23) ;
- une chambre de traitement (30) disposée entre la chambre d'anode (10) et la chambre de cathode (20) et comportant deux ouvertures (31, 32) et un échangeur de cations, la chambre d'anode (10) et la chambre de cathode (20) étant respectivement séparées de la chambre de traitement (30) par l'intermédiaire d'une membrane permsélective perméable aux cations (33) ; et
- une source d'énergie (40) en liaison active avec l'anode (13) et la cathode (23) ;
dans lequel
- une des ouvertures (31 ; 32) de la chambre de traitement (30), les ouvertures (11, 12) de la chambre d'anode (10) et l'une des ouvertures (21 ; 22) de la chambre de cathode (20) sont reliées entre elles, de sorte que la chambre de traitement (30) est en liaison active hydrauliquement avec la chambre d'anode (10) et que la chambre d'anode (10) est en liaison active hydrauliquement avec la chambre de cathode (20), permettant ainsi à un échantillon liquide de s'écouler au moins partiellement à travers la chambre de traitement remplie au moins en partie du échangeur de cations, puis de s'écouler au moins partiellement à travers la chambre d'anode, et puis de s'écouler au moins partiellement à travers la chambre de cathode, **caractérisé en ce que**
ladite ouverture (31 ; 32) de la chambre de traitement (30), les ouvertures (11, 12) de la chambre d'anode (10) et ladite ouverture (21 ; 22) de la chambre de cathode (20) sont reliées entre elles, de sorte que l'échantillon liquide fourni est guidé dans la chambre de traitement (30) dans le sens de la gravité et dans la chambre d'anode (10) et la chambre de cathode (20) dans le sens contraire à la gravité.

3. Dispositif (1) selon l'une des revendications 1 à 2, dans lequel un capteur de conductivité (51) est agencé avant les ouvertures non reliées (31 ; 32) de la chambre de traitement (30).

4. Dispositif (1) selon l'une des revendications 1 à 3, dans lequel un capteur de conductivité (52) est agencé entre la chambre de traitement (30) et la chambre d'anode (10).

5. Dispositif (1) selon la revendication 4, comprenant une unité de dégazage (41) agencée après le capteur de conductivité (52), un autre capteur de conductivité (53) étant agencé après l'unité de dégazage (41).

6. Dispositif (1) selon l'une des revendications 3 à 5, dans lequel au moins l'un des capteurs de conductivité (51 ; 52 ; 53) comprend une sonde de température.

7. Dispositif (1) selon l'une des revendications 1 à 6, comprenant au moins un capteur de débit agencé avant au moins l'un des éléments suivants :
- une des ouvertures (31 ; 32) de la chambre de traitement (30) ;
- une des ouvertures (11 ; 12) de la chambre d'anode (10)
- une des ouvertures (21 ; 22) de la chambre de cathode (20).

8. Dispositif (1) selon l'une des revendications 1 à 7, dans lequel l'échangeur d'anions est un échangeur d'anions indicateur de couleur ou dans lequel l'échangeur de cations est un échangeur de cations indicateur de couleur.

9. Dispositif (1) selon l'une des revendications 1 à 8, dans lequel la chambre de traitement (30) est réalisée au moins en partie transparente, en particulier le long des ouvertures (31, 32) de la chambre de traitement (30), le dispositif comprenant de préférence un capteur optique pour surveiller l'échangeur d'anions ou l'échangeur de cations.

10. Dispositif (1) selon l'une des revendications 3 à 9, comprenant une électronique de mesure qui capture et traite au moins l'un des éléments suivants :
- signal d'au moins l'un des capteurs de conductivité
- signal d'au moins l'une des sondes de température
- signal d'au moins l'un des capteurs de débit
- signal du au moins un capteur optique
- une tension de la source d'énergie (40)
- un courant de la source d'énergie (40).

11. Dispositif (1) selon l'une des revendications 1 à 10, comprenant au moins une membrane conductrice d'ions, agencée dans au moins l'une des chambres suivantes :
- chambre d'anode (10) entre l'anode (13) et la membrane permsélective orientée vers l'anode (13)
- chambre de cathode (20) entre la cathode (23) et la membrane permsélectives (33) orientée vers la cathode (23).

12. Dispositif (1) selon l'une des revendications 1 à 11, dans lequel la chambre de traitement (30) est interchangeable et/ou dans lequel au moins l'une des ouvertures (31, 32) de la chambre de traitement (30) comprend une unité de filtre.

13. Dispositif selon l'une des revendications 1 à 12, dans lequel la chambre de traitement (30) est interchangeable et présente une structure de base en forme de parallélépipède, comportant deux ouvertures (31, 32) espacées l'une de l'autre et deux surfaces latérales opposées formées chacune par une membrane permsélective correspondante (33) qui viennent recouvrir totalement l'espace vide formé par le cadre, dans lequel les surfaces latérales opposées comprennent chacune un cadre rectangulaire, formant une partie de la structure de base, sur lequel une membrane permsélective (33) est appliquée de manière adhésive et étanche aux gaz et à l'eau, et dans lequel le cadre présente une rainure.

14. Dispositif selon l'une des revendications 1 à 12, dans lequel la chambre de traitement (30) est interchangeable et présente une structure de base en forme de parallélépipède, comportant deux ouvertures (31, 32) espacées l'une de l'autre et deux surfaces latérales opposées formées chacune par une membrane permsélective perméable aux anions (33) dans le cas d'un échangeur d'anions ou par une membrane permsélective perméable aux cations (33) dans le cas d'un échangeur de cations, dans lequel les surfaces latérales opposées comprennent chacune un cadre rectangulaire, formant une partie de la structure de base, sur lequel la membrane permsélective (33) est appliquée de manière adhésive, et dans lequel est aménagée, de façon adjacente à l'une des ouvertures (31 ; 32) une autre ouverture pour remplir l'échangeur d'anions ou l'échangeur de cations, et/ou une autre ouverture pour le dégazage, de préférence au moins l'une des autres ouvertures pouvant être fermée de façon étanche à l'eau et au gaz.

15. Dispositif selon l'une des revendications 1 à 12, dans lequel la chambre de traitement (30) est interchangeable et présente une structure de base en forme de parallélépipède, comportant deux ouvertures (31, 32) espacées l'une de l'autre et deux surfaces latérales opposées formées chacune par une membrane permsélective (33), dans lequel les surfaces latérales opposées comprennent chacune un cadre rectangulaire, formant une partie de la structure de base, sur lequel est appliquée de façon adhésive la membrane permsélective (33), et dans lequel les côtés extérieurs des surfaces latérales formées par la membrane permsélective (33) sont munis d'éléments de protection amovibles, en particulier de plaques rigides.

16. Procédé pour l'électrodéionisation d'un échantillon liquide à mettre en oeuvre dans un dispositif selon la revendication 2, comprenant les étapes consistant à :
- appliquer une tension à une anode (13) et une cathode (23) séparées dans l'espace par deux membranes permsélectives perméables aux cations (33a, 33b) ;
- faire passer au moins partiellement l'échantillon liquide à travers une chambre de traitement (30) remplie au moins en partie d'un échangeur de cations et délimitée par les deux membranes permsélectives perméables aux cations (33a, 33b) ; puis
- faire passer au moins partiellement l'échantillon liquide à travers une chambre d'anode (10) située entre l'anode (13) et une des membranes permsélectives perméables aux cations (33a), puis
- faire passer au moins partiellement l'échantillon liquide à travers une chambre de cathode (20) située entre la cathode (23) et l'autre membrane permsélective perméable aux cations (33b),
**caractérisé en ce que**
- l'écoulement au moins partiel à travers la chambre de traitement (30) remplie au moins en partie d'un échangeur de cations se fait dans le sens de la gravité ;
- l'écoulement au moins partiel à travers la chambre d'anode (10) se fait dans le sens contraire à la gravité ; et
- l'écoulement au moins partiel à travers la chambre de cathode (20) se fait dans le sens contraire à la gravité.

17. Procédé pour l'électrodéionisation d'un échantillon liquide à mettre en oeuvre dans un dispositif selon la revendication 1, comprenant les étapes consistant à :
- appliquer une tension à une anode (13) et une cathode (23) séparées dans l'espace par deux membranes permsélectives perméables aux anions (33a, 33b) ;
- faire passer au moins partiellement l'échantillon liquide à travers une chambre de traitement (30) remplie au moins en partie d'un échangeur d'anions et délimitée par les deux membranes permsélectives perméables aux anions (33a, 33b) ; puis
- faire passer au moins partiellement l'échantillon liquide à travers une chambre de cathode (20) située entre la cathode (23) et l'autre membrane permsélective perméable aux anions (33b) ; puis
- faire passer au moins partiellement l'échantillon liquide à travers une chambre d'anode (10) située entre l'anode (13) et une des membranes permsélectives perméables aux anions (33a),
**caractérisé en ce que**
- l'écoulement au moins partiel à travers la chambre de traitement (30) remplie au moins en partie d'un échangeur d'anions se fait dans le sens de la gravité ;
- l'écoulement au moins partiel à travers la chambre d'anode (10) se fait dans le sens contraire à la gravité ; et
- l'écoulement au moins partiel à travers la chambre de cathode (20) se fait dans le sens contraire à la gravité.

18. Procédé selon l'une des revendications 16 à 17, comprenant au moins l'une des étapes supplémentaires suivantes consistant à :
- mesurer la conductivité spécifique de l'échantillon liquide avant l'étape consistant à faire passer au moins partiellement l'échantillon liquide à travers la chambre de traitement (30) remplie au moins en partie de l'échangeur d'anions ou de l'échangeur de cations et délimitée par les deux membranes permsélectives (33a, 33b) ;
- mesurer la conductivité spécifique de l'échantillon liquide
après l'étape consistant à faire passer au moins partiellement l'échantillon liquide à travers la chambre de traitement (30) remplie au moins en partie avec l'échangeur d'anions ou l'échangeur de cations et délimitée par les deux membranes permsélectives (33a, 33b), en particulier en combinaison avec les autres étapes consistant à dégazer au moins une partie de l'échantillon liquide, et à mesurer ensuite une nouvelle fois la conductivité spécifique
après l'étape consistant à mesurer la conductivité spécifique de l'échantillon liquide ;
- mesurer de façon continue et intégrée le débit de l'échantillon liquide.
